# EUROPEAN PATENT SPECIFICATION

(11) **EP 3 651 819 B1**
(45) Date of publication and mention of the grant of the patent: **09.03.2022**
(21) Application number: 18708197.1
(22) Date of filing: 10.01.2018
(51) Int. Cl.: A61M 1/00

(54) **LIPOSUCTION DEVICE**
FETTABSAUGVORRICHTUNG
DISPOSITIF DE LIPOSUCCION

(30) Priority: 10.07.2017 IT 201700077603
(43) Date of publication of application: 20.05.2020
(73) Proprietor: Clover Orthopedics S.r.l., 20151 Milano (IT)
(72) Inventor: COLTELLINI, Leonardo, 06012 Città di Castello (PG) (IT); MINIERO, Marco, 47891 Dogana (SM); SABRY, Christian, 20851 Lissone (MB) (IT)
(74) Representative: Roncuzzi, Davide
(86) International application number: PCT/IB2018/050151
(87) International publication number: WO 2019/012337

(56) References cited:
- WO-A2-2012/006587
- US-A1- 2014 276 384
- US-A1- 2016 106 889
- US-B1- 9 581 942

## Description

The present invention relates to a liposuction device. In particular, the present invention relates to a device for extracting and separating lipoaspirated material. In more detail, the present invention relates to a device of the mentioned type comprising a bag and a filter housed inside the bag, wherein the invention comprises an innovative solution for discharging and/or cleaning said filter.

### BACKGROUND TO THE INVENTION

Liposuction is a known and widely used technique in the field of cosmetic surgery. On the other hand, in the field of general medicine, it is also known the use of the material resulting from liposuction (herein also referred to as "lipoaspirated" or "liposucked" material) for regenerating tissues and/or articulations.

To this end, the lipoaspirated material is collected in a separation bag provided with a filter for separating impurities (for example, blood and/or oil components) from "valuable" components such as adipose tissue to be used for regenerating tissues and/or articulations.

In particular, devices (simply called bags) are known and used for the above purposes, which are substantially provided with a main bag made of transparent and soft and/or malleable material, inside which a filter is arranged. The main bag also comprises a vent (not connected to the filter) and an outlet (also separate from, and therefore not connected to the filter). The filter comprises an inlet or opening for the aspirated material entering the filter and therefore the main bag. Examples of such systems are disclosed in documents US2010279405 and US2016106889 as well as the device registered by Meika Group SRL at the Italian national register of medical devices marked in Italy under the registration number 1473321.

The use of the known bags provides for the aspirated material entering the filter through the opening or valve thereof, wherein the material is then filtered, and wherein the impurities (such as blood and/or oil components) in the aspirated material pass through the filter and accumulate in the main bag outside the filter, while the adipose tissue is kept inside the filter. The known bags therefore allow to separate the adipose tissue from the impurities mentioned above.

The prior art bags are valuable from many viewpoints (for example effectiveness of separation), but have several drawbacks that are not yet addressed and that the Applicant wants to overcome or at least to reduce with the present invention.

For example, a first drawback or problem occurring while using the prior art bags is due to the fact that the bags do not allow directly to empty the filter, as for emptying the filter it is necessary to remove it from the bag.

The fact that the filter must be necessarily removed from the bag for being emptied means that it is impossible to use the prior art bags continuously, as with those bags it is necessary periodically to interrupt the operation for emptying the filter.

Moreover, in the case of prior art bags, it is necessary to remove the filter from the main bag also when it is only necessary to wash the filter, wherein washing the filter is necessary or at least suggested in case of treatment in succession, with the bag, of material aspirated from different patients.

The main object of the invention is to overcome the drawbacks of the prior art bags mentioned above.

In particular, one object of the invention is to allow to empty the filter without removing it from the main bag.

A further object of the present invention is to allow to wash the filter in a reliable and accurate manner, for example by inserting physiological saline and/or disinfectant and/or a similar solution into the filter without the need for removing it from the main bag.

In view of what above, it is clearly apparent that the achievement of the objects mentioned above allows the continuous use of the bag, with clear advantages in terms of time and cost saving.

### SUMMARY OF THE PRESENT INVENTION

The present invention is based on the consideration that the objects mentioned above can be effectively achieved by providing the filter with an outlet, through which the inside of the filter is connected to the outside of the bag. In fact, in this way it is possible to empty the filter by making the material contained herein exit through the outlet and therefore without the need for removing the filter from the main bag.

The same applied for washing the filter, wherein the washing solution inserted in the filter and full of impurities can be discharged through the same outlet.

In view of both the objects summarized above and the concept on which the invention is based, the present invention relates to a device for extracting and separating lipoaspirated material, said device comprising a main bag and a filter housed inside said main bag; wherein said device comprises a first inlet valve, through which the inside of said filter is connected to the outside of said main bag for inserting said lipoaspirated material into said main bag through said filter; said device comprising a second outlet or emptying valve, through which the inside of said filter is hydraulically connected to the outside of said main bag for directly discharging or emptying said filter.

According to an embodiment, said device comprises a third outlet or emptying valve, through which the inside of said main bag is hydraulically connected to the outside for directly discharging or emptying said main bag.

According to an embodiment, said device comprises a fourth air valve, through which the inside of said main bag is connected to the outside for blowing air out of said main bag.

According to the invention said filter is tubular.

According to an embodiment, the pore size of said filter is 50 µm.

According to an embodiment, said main bag is made of transparent and malleable material.

According to an embodiment, said device comprises a substantially rigid box-shaped body, inside which said main bag is housed; said body is provided with at least one access window in correspondence of said filter.

According to an embodiment, said device comprises emptying means that are fixed to said box-shaped body and suitable to apply pressure to said filter.

According to an embodiment, said emptying means comprise a spatula that can translate along said at least one access window, wherein the translation of said spatula along said at least one access window pressing on said filter results in a push on the content of the filter towards said second outlet or emptying valve and therefore in said content exiting from said filter through said second outlet or emptying valve.

According to an embodiment, at least one of said first inlet valve, second outlet or emptying valve and third outlet or emptying valve is a Luer lock valve equipped with a silicone membrane that can be opened as a syringe tip or other similar element is inserted.

### BRIEF DESCRIPTION OF DRAWINGS

Further characteristics and advantages of the device according to the present invention will be more apparent from the description below of non-limiting embodiments of the device according to the invention, illustrated in the drawing. In the drawing, identical or corresponding parts and/or features of the device according to the embodiments illustrated are identified by the same reference numbers. In particular, in the drawing:
- figure 1 is a front view of an embodiment of the device according to the present invention;
- figure 2 is a front view, with some parts removed for the sake of clarity, of a detail from figure 1 in enlarged scale; and
- figure 3 is a side view of figure 1, in enlarged scale and with some parts removed for the sake of clarity.

### DETAILED DESCRIPTION OF THE PRESENT INVENTION

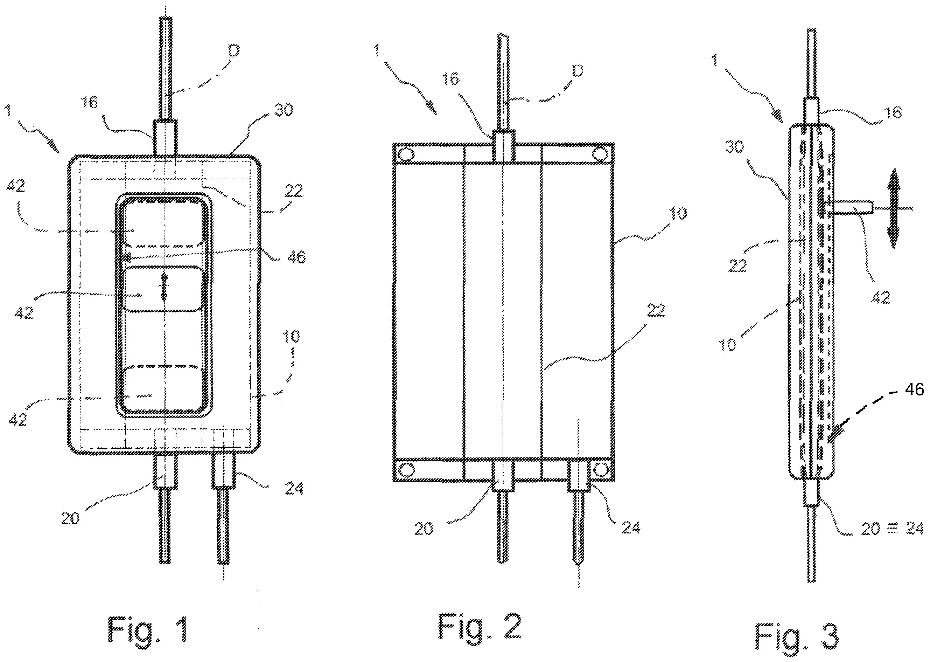

In Figs. 1-3, number 1 indicates, as a whole, a device according to and embodiment of the present invention. As shown, the device 1 comprises a main bag 10 (whose use will be described in detail below) housed in a box-shaped container 40 made of a substantially rigid material. The box-shaped container 50 is provided with an access window 46. It should be specified that the container 40 is not essential for the main purposes of the present invention; the container 40 shall be therefore deemed as a non-essential fitting, the main features of the device according to the invention being related to the filter 22 housed in the main bag 10. The description below of the above mentioned features is independent of the presence of the container 40, the advantages provided by the container 40 being described below separately.

As mentioned, a filter 22 is housed in the main bag 10 made for example of a transparent and/or soft and/or malleable material of the type used of containing physiological materials, the filter being also malleable and being tubular in shape. To a first end of the filter 22 a first inlet valve 16 is applied, which extends through the main bag 10 and through which the inside of the filter 22 is connected to the outside of the main bag 10, sealing means being provided in correspondence of the first valve 16, in particular between the first valve 16 and the bag 10, in order to avoid undesired lacks of material. To this end, the bag 10 may be for example heat-welded or glued to the outside of the first valve 16.

To the second end of the filter 22, opposite to the first end, a second outlet valve 20 is applied, which extends through the bag 10 and through which the inside of the filter 22 is connected to the outside of the main bag 10. In this case again, sealing means may be provided in correspondence of the second valve 20, in order to avoid undesired lacks of material, wherein, in this case again, the bag 10 may be for example heat-welded or glued to the outer surface of the second outlet valve 20.

According to the embodiment illustrated in the figures, the device 1 further comprises a third outlet valve 24 connecting the inside of the bag 10 to the outside of the bag, sealing means like those described above being provided in correspondence of the third outlet valve 24. It should be specified that the space inside the bag 10 and outside the filter 22 is connected with the outside through the third outlet valve 24. Lastly, a fourth air valve is also provided, not shown in the figures, through which air is blowing out from the inside of the main bag 10 towards the outside of the main bag 10.

The use of the device according to the embodiment described above, and therefore not including the box-shaped container 40, can be summarized as follows. the material to be treated, in particular resulting from liposuction, is inserted into the bag 10 through the first inlet valve 16, wherein the material is firstly inserted into the filter 22. The action of the filter 22, that has for example, although without limitation, a pore size equal to or greater than 50 µm, results in the separation of the blood and/or oil components from the adipose components; in particular, the blood and/or oil components, that are thinner, passing through the filter 22 and spread in the bag 10 outside the filter 22, whilst the adipose components are kept inside the filter 22. While inserting the lipoaspirated material according to what described above, the air in the bag 10, or inserted into the air 10, exits from the bag 10 through the third outlet valve 24. Once the bag 10 is completely filled, or once the desired level has been achieved, the bag 10 can be emptied making the blood and/or oil components exit through the third valve 24. Moreover, the second outlet valve 20, that, as mentioned above, directly connects the inside of the filter 22 to the outside of the bag 10, allows to empty the filter 22, without the need for removing the filter 22 from the bag 10, and therefore exactly according to the objects defined at the beginning. To this end, through a simple tool, substantially shaped, for example, like a spatula, by pressing the filter 22 and moving the spatula along the filter 22, the material contained inside it (the adipose material without the blood and/or oil components) can be pushed towards the second outlet valve 20 and therefore forced to exit through it substantially in the same manner as occurs for emptying a tube of toothpaste.

Similarly, the filter 22 can be washed effectively by inserting a washing solution through the first valve 16 and discharging it through the second valve 20 and/or the third valve 24, in this case again without the need for removing the filter from the main bag 10.

Now, the embodiment of the inventive device comprising the box-shaped container 40 will be described below with reference to the figures.

Obviously, in correspondence of each of the valves 16, 20, 24 and of the air valve, the container 40 has a corresponding orifice through which the respective valve extends. Moreover, in correspondence of the filter 22, the container 40 has a window 46, whose longitudinal extension is substantially parallel to the longitudinal extension D of the filter 22. The container 40 therefore ensure an adequate protection of the bag 40 and allows to stack more devices 1 over one another, wherein the window 46 allows to engage, for example through a spatula 42, the filter 22 according to that summarized above, without the need for removing the bag 10 from the container 40. Moreover, as shown in the figures, the spatula 42 may be fastened to the container 40 so that it can be translated along the window 46 and, if necessary, so that it can be switched through rotation around a rotation axis perpendicular to the direction of longitudinal extension of the window 46. The detailed description below of the embodiments of the invention illustrated in the figures clearly proved that the invention allows to overcome the drawbacks of the prior art bags for extracting and separating lipoaspirated material. In particular, the present invention allows both to empty and wash the filter 22 without the need for removing the filter 22 from the bag 10 where it is housed. Even if the device according to the invention has been explained in the above detailed description of the embodiments shown in the drawing, however the present invention is not limited to the embodiments described above and illustrated in the figures. Contrariwise, all variants and modifications of the embodiments described and illustrated in this document that are clearly apparent to those skilled in the art fall within the scope of the invention. For example, the valves 16, 20, 24 and the air valve, known and not shown, may be, according to the needs and/or circumstances, 2-way or more-way valves, or valves comprising a membrane, for example a silicone membrane, that can be opened as a syringe tip or other similar element is inserted. Moreover, on the bag 10 and/or on the container 40 means may be provided for respectively hanging the bag 10 or the container 40, for example to a support trestle, according to modes substantially similar to those used for hanging a bag or container for I.V. drip.

The scope of the present invention is defined by the appended claims.

## Claims

1. Device (1) for extracting and separating lipoaspirated material.; said device (1) comprising a main bag (10) and a filter (22) housed inside said main bag (10); wherein said device comprises a first inlet valve (1.6) which extends through the main bag (10) and through which the inside of said filter (22) is connected to the outside of said main bag (10) for inserting said lipoaspirated material into said main bag (10) through said filter (22); wherein said device (1) comprises a second outlet or emptying valve (20) which extends through said main bag (10) and through which the inside of said filter (22) is hydraulically connected to the outside of said main bag (10) for directly discharging or emptying said filter (22); wherein said device comprises a third outlet or emptying valve (24), through which the inside of said main bag (10) is hydraulically connected to the outside for directly discharging or emptying said main bag (10) **characterized by** the filter being tubular in shape.

2. Device according to claim 1, **characterized by** comprising a fourth air valve, through which the inside of said main bag (10) is connected to the outside for blowing air out of said main bag (10).

3. Device according to any one of the preceding claims, **characterized in that** the pore size of said filter (22) is 50 µm.

4. Device according to any one of the preceding claims, **characterized in that** said main bag (10) is made of transparent and malleable material.

5. Device according to any one of claims 1 to 4, **characterized by** comprising a substantially rigid box-shaped body (30), inside which said main bag (10) is housed; said body (30) being provided with at least one access window (46) in correspondence of said filter (22) .

6. Device according to claim 5, **characterized by** comprising emptying means (40) that are fixed to said box-shaped body (30) and suitable to apply pressure to said filter (22) .

7. Device according to claim 6, **characterized in that** said emptying means (40) comprise a spatula (42) that can translate along said at least one access window (46), wherein the translation of said spatula (42) along said at least one access window (46) pressing on said filter (22) results in a push on the content of the filter (22) towards said second outlet or emptying valve (20) and therefore in said content exiting from said filter (22) through said second outlet or emptying valve (20).

8. Device according to any one of claims 1 to 7, **characterized in that** at least one of said first inlet valve (16), second outlet or emptying valve (20) and third outlet or emptying valve (24) is a Luer lock valve equipped with a silicone membrane that can be opened as a syringe tip or other similar element is inserted.

## Patentansprüche

1. Vorrichtung (1) zum Extrahieren und Trennen von lipoaspiriertem Material; wobei die Vorrichtung (1) einen Hauptbeutel (10) und einen Filter (22) umfasst, die innerhalb des Hauptbeutels (10) untergebracht ist; wobei die Vorrichtung ein erstes Einlassventil (16) umfasst, das sich durch den Hauptbeutel (10) erstreckt und durch das das Innere des Filters (22) mit der Außenseite des Hauptbeutels (10) verbunden ist, um das lipoaspirierte Material durch den Filter (22) in den Hauptbeutel (10) einzubringen; wobei die Vorrichtung (1) ein zweites Auslass- oder Entleerungsventil (20) umfasst, das sich durch den Hauptbeutel (10) erstreckt und durch das das Innere des Filters (22) hydraulisch mit der Außenseite des Hauptbeutels (10) verbunden ist für direktes Ausgeben oder Entleeren des Filters (22); wobei die Vorrichtung ein drittes Auslass- oder Entleerungsventil (24) umfasst, durch welches das Innere des Hauptbeutels (10) hydraulisch mit der Außenseite verbunden ist, um den Hauptbeutel (10) direkt auszulassen oder zu entleeren, **dadurch gekennzeichnet, dass** der Filter eine rohrförmige Form aufweist.

2. Vorrichtung nach Anspruch 1, **dadurch gekennzeichnet, dass** sie ein viertes Luftventil umfasst, durch das das Innere des Hauptbeutels (10) mit der Außenseite verbunden ist, um Luft aus dem Hauptbeutel (10) auszublasen.

3. Vorrichtung nach einem der vorhergehenden Ansprüche, **dadurch gekennzeichnet, dass** die Porengröße des Filters (22) 50 µm beträgt.

4. Vorrichtung nach einem der vorhergehenden Ansprüche, **dadurch gekennzeichnet, dass** der Hauptbeutel (10) aus transparentem und formbarem Material besteht.

5. Vorrichtung nach einem der Ansprüche 1 bis 4, **dadurch gekennzeichnet, dass** sie einen im Wesentlichen steifen kastenförmigen Körper (30) umfasst, in dem der Hauptbeutel (10) untergebracht ist; wobei der Körper (30) mit mindestens einem Zugangsfenster (46) in Übereinstimmung mit dem Filter (22) versehen ist.

6. Vorrichtung nach Anspruch 5, **dadurch gekennzeichnet, dass** sie Entleerungsmittel (40) umfasst, die an dem kastenförmigen Körper (30) befestigt sind und geeignet sind, Druck auf den Filter (22) auszuüben.

7. Vorrichtung nach Anspruch 6, **dadurch gekennzeichnet, dass** die Entleerungsmittel (40) einen Spatel (42) umfassen, der sich entlang des mindestens einen Zugangsfensters (46) bewegen kann, wobei die Translation des Spatels (42) entlang des mindestens einen Zugangsfensters (46), das auf den Filter (22) drückt, dazu führt, dass der Inhalt des Filters (22) in Richtung des zweiten Auslass- oder Entleerungsventils (20) gedrückt wird und daher der Inhalt aus dem Filter (22) durch das zweite Auslass- oder Entleerungsventil (20) austritt.

8. Vorrichtung nach einem der Ansprüche 1 bis 7, **dadurch gekennzeichnet, dass** mindestens eines von dem ersten Einlassventil (16), dem zweiten Auslass oder dem Entleerungsventil (20) und dem dritten Auslass oder Entleerungsventil (24) ein Luer-Lock-Ventil ist, das mit einer Silikonmembran ausgestattet ist, die als Spritzenspitze geöffnet werden kann oder ein ähnliches Element eingesetzt wird.

## Revendications

1. Dispositif (1) pour extraire et séparer de la matière aspirée par liposuccion ; ledit dispositif (1) comprenant un sac principal (10) et un filtre (22) reçu à l'intérieur dudit sac principal (10) ; ledit dispositif comprenant une première valve d'entrée (16) qui s'étend à travers le sac principal (10) et par laquelle l'intérieur dudit filtre (22) est relié à l'extérieur dudit sac principal (10) pour introduire ladite matière aspirée par liposuccion dans ledit sac principal (10) à travers ledit filtre (22) ; ledit dispositif (1) comprenant une deuxième valve de sortie ou de vidange (20) qui s'étend à travers ledit sac principal (10) et par laquelle l'intérieur dudit filtre (22) est relié hydrauliquement à l'extérieur dudit sac principal (10) pour directement décharger ou vider ledit filtre (22) ; ledit dispositif comprenant une troisième valve de sortie ou de vidange (24), par laquelle l'intérieur dudit sac principal (10) est relié hydrauliquement à l'extérieur pour directement décharger ou vider ledit sac principal (10), **caractérisé par le fait que** le filtre est de forme tubulaire.

2. Dispositif selon la revendication 1, **caractérisé par le fait qu'**il comprend une quatrième valve d'air, par laquelle l'intérieur dudit sac principal (10) est relié à l'extérieur pour souffler de l'air hors dudit sac principal (10) .

3. Dispositif selon l'une quelconque des revendications précédentes, **caractérisé par le fait que** la dimension de pore dudit filtre (22) est de 50 µm.

4. Dispositif selon l'une quelconque des revendications précédentes, **caractérisé par le fait que** ledit sac principal (10) est fait de matériau transparent et malléable.

5. Dispositif selon l'une quelconque des revendications 1 à 4, **caractérisé par le fait qu'**il comprend un corps en forme de boîte sensiblement rigide (30), à l'intérieur duquel est reçu ledit sac principal (10) ; ledit corps (30) comportant au moins une fenêtre d'accès (46) en correspondance avec ledit filtre (22).

6. Dispositif selon la revendication 5, **caractérisé par le fait qu'**il comprend des moyens de vidange (40) qui sont fixés audit corps en forme de boîte (30) et appropriés pour appliquer une pression audit filtre (22).

7. Dispositif selon la revendication 6, **caractérisé par le fait que** lesdits moyens de vidange (40) comprennent une spatule (42) qui peut se déplacer en translation le long de ladite au moins une fenêtre d'accès (46), la translation de ladite spatule (42) le long de ladite au moins une fenêtre d'accès (46) appuyant sur ledit filtre (22) résultant en une poussée sur le contenu du filtre (22) vers ladite deuxième valve de sortie ou de vidange (20) et, de ce fait, en une sortie dudit contenu dudit filtre (22) par ladite deuxième valve de sortie ou de vidange (20).

8. Dispositif selon l'une quelconque des revendications 1 à 7, **caractérisé par le fait qu'**au moins l'une parmi ladite première valve d'entrée (16), ladite deuxième valve de sortie ou de vidange (20) et ladite troisième valve de sortie ou de vidange (24) est une valve Luer lock comportant une membrane en silicone qui peut être ouverte lorsqu'une pointe de seringue ou un autre élément similaire est introduit.
